# EUROPEAN PATENT SPECIFICATION

(11) **EP 0 912 200 B1**
(45) Date of publication and mention of the grant of the patent: **10.12.2003**
(21) Application number: 97926497.5
(22) Date of filing: 14.05.1997
(51) Int. Cl.: A61L 9/12

(54) **PLASTIC ENVELOPE ASSEMBLY AIR FRESHENER DISPENSER DEVICE**
KUNSTOFFHÜLLE-LUFTVERBESSERUNGSVORRICHTUNG
DISPOSITIF DISTRIBUTEUR DE DESODORISANT D'AMBIANCE A ENVELOPPE PLASTIQUE

(30) Priority: 14.05.1996 US 645977
(43) Date of publication of application: 06.05.1999
(73) Proprietor: S.C. Johnson & Son, Inc., Racine, WI 53403-2236 (US)
(72) Inventor: MARTIN, John, Racine, WI 53402 (US); ROSPLOCK, Joseph, M., Racine, WI 53402 (US)
(74) Representative: Carpmaels & Ransford
(86) International application number: US9708164
(87) International publication number: WO97042983

(56) References cited:
- EP-A- 0 081 791
- EP-A- 0 093 262
- WO-A-84/02654
- WO-A-95/15772
- WO-A-97/22370
- FR-A- 1 316 894
- GB-A- 2 123 352
- US-A- 3 343 664

## Description

### Technical Field

This invention generally relates to dispensers of vaporizable media. More specifically, this invention relates to a device for dispensing a fragrance or deodorant in the form of a vapor for air freshening in an enclosed environment.

### Background Art

The need for effectively combating airborne malodors in homes and enclosed public buildings, by odor masking or destruction, is well established. Various kinds of vapor-dispensing devices have been employed for this purpose. The most common of such devices is the aerosol container which propels minute droplets of an air freshener composition into the air. Another common type of dispensing device is a dish containing or supporting a body of gelatinous matter which when it dries and shrinks releases a vaporized air-treating composition into the atmosphere. Other products such as deodorant blocks and liquid wicks are also used for dispensing air-treating vapors into the atmosphere by evaporation. Another group of vapor-dispensing devices utilizes a carrier material such as paperboard impregnated or coated with a vaporizable composition.

A number of recent developments include a liquid air-treating composition in an enclosure, all or part of which is formed of a polymeric film through which the air-treating composition can migrate to be released as a vapor at an outer surface. Use of this type of permeable polymeric membrane controls the dispensing of air-treating vapors and tends to eliminate great variations in the rate of dispensing over the life of the product. Such products are considered advantageous when compared with the many air-treating products for which the rate of vapor release drops substantially over the life of the product.

Products of the type having a sheet of permeable polymeric material to control the emission of air-treating vapors may be in a variety of forms. In some, the polymeric sheet covers a cylindrical container, while in others the liquid air-treating material is trapped between the permeable sheet and an impermeable plastic sheet. In still others, the permeable polymeric material forms a flexible pouch having a content of the air-treating liquid. The liquid, prior to activation, is isolated within a breakable container such as a glass vial or an impermeable plastic inner pouch, or the like.

Publications of background interest in connection with air freshener devices include United States Patent Numbers 2,481,296; 2,594,714; 3,785,556; 3,790,081; 3,946,945; 4,130,245; 4,220,281; 4,306,679; 4,382,548; 4,502,630; 4,558,820; 4,583,686; 4,615,486; 4,660,763; 4,630,775; 4,739,928; 4,849,606; 4,948,047; 4,595,925; 4,948,047; 4,960,240; 4,983,578; 4,998,671; and the like.

WO95/15772 discloses an air freshener dispenser comprising a container formed from two independent, multi-layered, flexible sheets which are sealed and contain a cotton-based wicking material and air-freshening composition.

Some air freshener dispensers are expensive to manufacture. Other air freshener dispensers are inexpensive to produce, but tend to have inferior construction and functionality.

There remains a need for a well-constructed air freshener dispenser device which can be mass-produced economically and which can deliver a vapor medium at a controlled uniform rate over an extended period of time.

Accordingly, it is an object of this invention to provide an improved air freshener dispenser device for delivering an odorant and/or deodorant vapor in an enclosed environment.

It is another object of this invention to provide an air freshener dispenser device with a primary structure that is a plastic envelope assembly composed of thin membrane laminates which can be produced economically by a thermoforming means in a continuous process.

It is a further object of this invention to provide an elongated reservoir enclosure which has a volatile air freshener content and an internally disposed semi-rigid support member, and which is sandwiched and sealed between two translucent or transparent thin film permeable membranes.

Other objects and advantages of the present invention shall become apparent from the accompanying description and drawings.

### Brief Summary of the Invention

One or more objects of the present invention are accomplished by the provision of an air freshener dispenser device which is a plastic envelope assembly comprising:
(a) an elongated thin membrane which is juxtapositioned coextensively with a second elongated thin membrane; wherein the two membranes have dimensional correspondence, and are bonded along a peripheral margin that frames a central section of opposing membrane surfaces which form a reservoir enclosure; and wherein each membrane is permeable to a volatile medium in the reservoir enclosure;
(b) a volatile air freshener medium which is contained within the reservoir enclosure;
(c) a thin peelable impermeable membrane which is in a laminate formation with each respective permeable membrane to prevent volatilization of the air freshener ingredient through the permeable membrane from the reservoir enclosure; and
(d) a thin semi-rigid strip which is situated unattached within the reservoir enclosure, and has dimensions which closely approximate the area dimensions of the reservoir enclosure; wherein the strip functions as a stiffening means which imparts semi-rigidity to the dispenser article, and wherein the strip structure has at least one aperture which provides free access between the inner surfaces of the two permeable membranes, wherein, the semi-rigid strip is a moulded, thermoplastic structure.

### Description of the Drawings

FIG. 1 is a perspective view of an invention air freshener dispenser device with an imprinted design.
FIG. 2 is a sectional view taken along lines 2-2 of FIG. 1.
FIG. 3 is a perspective view of a FIG. 1 air freshener dispenser device which illustrates the removal of two peelable vapor-impermeable membranes.
FIG. 4 is a perspective view of a FIG. 3 air freshener dispenser device with the complete removal of two peelable vapor-impermeable membranes.
FIGS. 5-8, respectively, illustrate aperture configurations in a semi-rigid thermoplastic strip which is an insert in the reservoir enclosure of an invention air freshener as shown in FIG. 4.

### Detailed Description of the Invention

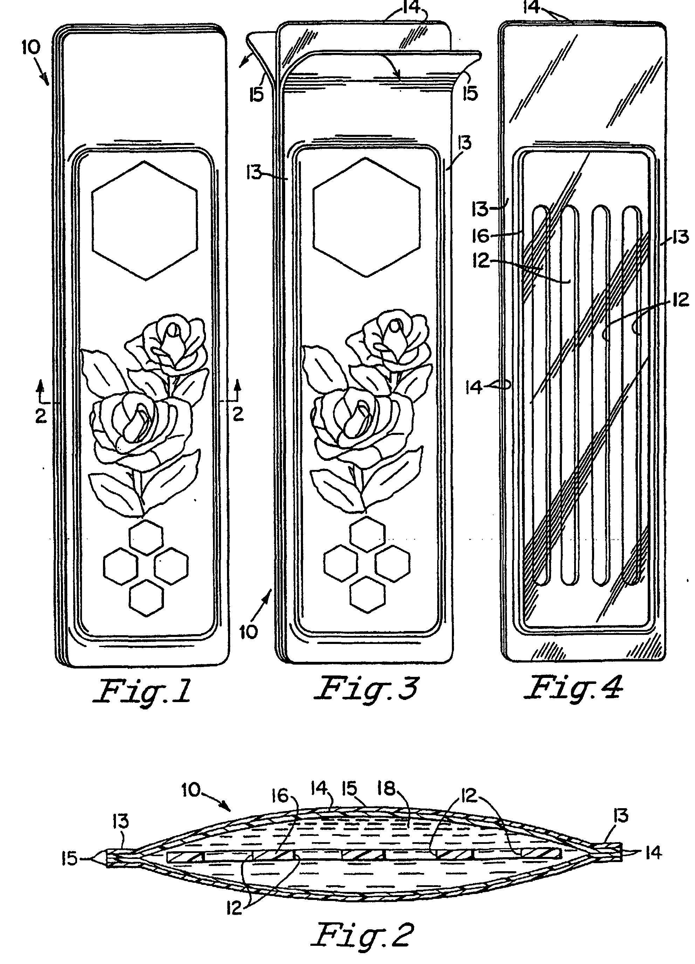

FIG. 1 illustrates a present invention air freshener dispenser device which has a design imprinted on its upper surface.

A FIG. 1 type of air freshener dispenser device has a semi-rigid structure, and its typical dimensions are about four inches in length, about one half to one inch in width, and about one eighth to one fourth inch in thickness.

A FIG. 1 type of air freshener dispenser device can be utilized by peeling one of the outer impermeable membranes partially or completely, or peeling the outer impermeable membrane from each side of the dispensing device.

FIG. 2 is a cross-sectional end view of a FIG. 1 type of air freshener dispenser device which has an elongated grating of parallel apertures 12 in semi-rigid strip 16. Membranes 14 and 15 are in a convex configuration to provide an increased capacity for air freshener medium 18. Thin film vapor-permeable membranes 14 and thin film vapor-impermeable membranes 15 are in a laminate formation, and membranes 14 and 15 are bonded along periphery margin 13. Semi-rigid strip 16 is unattached within the reservoir enclosure which contains air freshener medium 18.

FIG. 3 is a prospective view of air freshener dispenser device 10 of FIG. 1, in which two vapor-impermeable membranes 15 are being peeled from the surfaces of vapor-permeable membrane 14. Membranes 14 are bonded along peripheral margin 13.

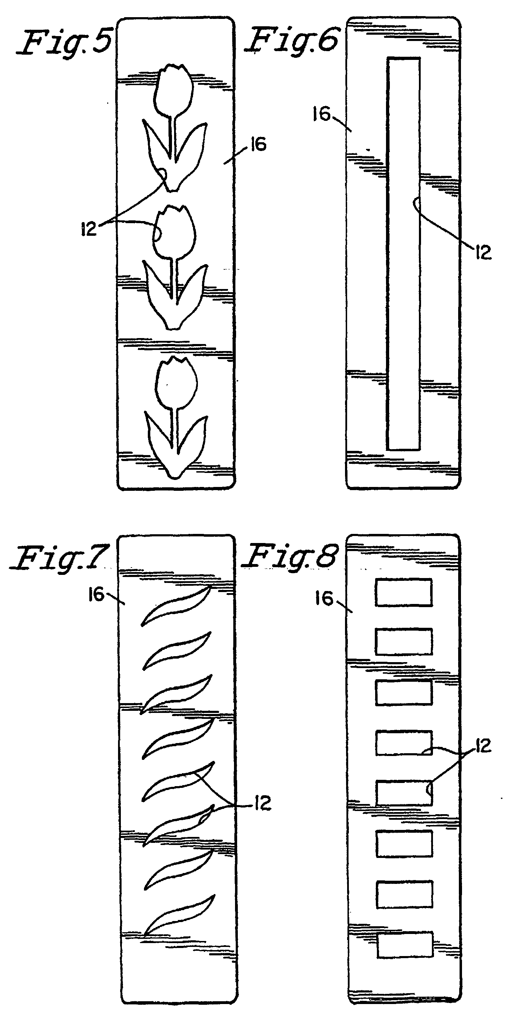

FIG. 4 is a perspective view of the air freshener dispenser device of FIG. 3, in which vapor-impermeable membranes 15 are completely removed. FIG. 4 illustrates air freshener dispenser device in which vapor-permeable membranes 14 are transparent. Semi-rigid strip 16 is visible through membranes 14, as is a reservoir content of air freshener medium which is not shown in FIG. 4. Apertures 12 in FIG. 4 correspond to grating apertures 12 illustrated in FIG. 2.

Other aperture 12 configurations are represented in FIGS. 5-8. Multiple apertures 12 provide a reservoir capacity for air freshener 18 as illustrated in FIG. 2. Multiple apertures 12 can be in the form of a design pattern, as illustrated in FIG. 5.

Thin film vapor-impermeable membrane 15 is bonded to vapor-permeable membrane 14 in the form of a laminate. Vapor-impermeable membrane 15 is peelable, so that its partial or complete removal allows air freshener medium 18 to migrate through vapor-permeable membrane 14 and volatilize into the atmosphere.

Semi-rigid strip 16 is provided by thermoform molding of a thermoplastic polymer such as polyethylene, polypropylene, polyvinyl chloride, and the like.

Vapor-permeable membrane 14 can be in the form of a flexible thin film of a thermoplastic polymer such as polyethylene, isotactic polypropylene, cellulose acetate, and the like. Membrane 14 permits migration of the enclosed volatile air freshener medium 18, either as a liquid or a vapor, depending on the type of membrane 14 being employed. Membrane 14 can be a microporous type (submicron pores), such as isotactic hydrophobic polypropylene film sold under the CELGARD tradename (Celanese). Microporous thermoplastic polymer films are described in U.S. 3,055,297.

Vapor-impermeable membrane 15 can be in the form of a flexible thin film such as aluminum foil or nylon film, which is peelable from its adhering bond to vapor-permeable membrane 14.

In a preferred embodiment, a laminate of membrane 14 and membrane 15 is preformed, and then two identical sections are juxtapositioned and formed into a present invention envelope type product which has a content of air freshener medium 18, and semi-rigid strip 16, in a sealed reservoir enclosure. Production of a laminate of permeable and impermeable membranes is illustrated in U.S. 4,145,001.

Air freshener ingredient 18 can be any air treating material which can migrate through membrane 14 and disperse into the atmosphere in vapor form. Typically air freshener ingredient 18 is a fragrance or a deodorant in liquid or gel form.

Preferably, air freshener ingredient 18 is a liquid fragrance comprising one or more volatile organic compounds which are available from perfumery suppliers such as Firmenich Inc., Takasago Inc., Noville Inc., Quest Co., and Givaudan-Roure Corp.

Most conventional fragrance materials are volatile essential oils. The fragrance can be a synthetically formed material, or a naturally derived oil such as oil of Bergamot, Bitter Orange, Lemon, Mandarin, Caraway, Cedar Leaf, Clove Leaf, Cedar Wood, Geranium, Lavender, Orange, Origanum, Petitgrain, White Cedar, Patchouli, Lavandin, Neroli, Rose absolute, and the like.

A wide variety of chemicals are known for perfumery, such as aldehydes, ketones, esters, alcohols, terpenes, and the like. A fragrance can be relatively simple in composition, or can be a complex mixture of natural and synthetic chemical components.

A typical scented oil can comprise woody/earthy bases containing exotic constituents such as sandalwood oil, civet, patchouli oil, and the like. A scented oil can have a light floral fragrance, such as rose extract or violet extract. Scented oil also can be formulated to provide desirable fruity odors, such as lime, lemon or orange.

Synthetic types of fragrance compositions either alone or in combination with natural oils are described in United States Patents 4,314,915; 4,411,829; and 4,434,306. Other artificial liquid fragrances include geraniol, geranyl acetate, eugenol, isoeugenol, linalool, linalyl acetate, phenethyl alcohol, methyl ethyl ketone, methylionone, isobornyl acetate, and the like.

A fragrance ingredient also can be in the form of a crystalline solid, which have the ability to sublime into the vapor phase at ambient temperatures. A crystalline fragrance starting material can be selected from organic compounds which include vanillin, ethylvanillin, coumarin, tonalid, calone, heliotropene, musk xylol, cedrol, musk ketone, benzophenone, raspberry ketone, methyl naphthyl ketone beta, phenyl ethyl salicylate, veltol, maltol, maple lactone, proeugenol acetate, evernyl, and the like. This type of fragrance can contribute a long term air-treating capability to an air freshener dispenser device.

In a further embodiment this invention provides a method for the manufacture of a claim 1 type of air freshener dispenser device, which comprises (1) providing two elongated thin membrane laminates of dimensional correspondence and with juxtapositional surfaces; wherein each laminate is composed of an inner vapor-permeable thin film, and a coextensive outer vapor-impermeable thin film; and wherein the two membrane laminates are bonded along a peripheral margin on three sides of the membrane laminates, thereby framing a central section of opposing membrane laminate surfaces and forming a reservoir enclosure; (2) inserting a thin semi-rigid strip through the unbonded open side of the two proximate membrane laminates, and into the reservoir enclosure; wherein the strip has dimensions which closely approximate the area dimensions of the reservoir enclosure; and wherein the strip structure has at least one aperture which provides free access between the inner surfaces of the two membrane laminates; and wherein the semi-rigid strip is a molded thermoplastic structure; (3) charging the reservoir enclosure with a volatile air freshener medium through the unbonded open side of the membrane laminates; and (4) bonding the open side of the membrane laminates to seal the volatile air freshener medium within the reservoir enclosure.

The two laminate sections with juxtapositioned flat surfaces typically are bonded along the peripheral margin by heat-sealing means. The inner vapor-permeable membranes of the two laminates are adapted to heat-seal with each other. The outer vapor-impermeable membranes of the two laminates are adapted to maintain the peelable property under the heat-sealing temperature conditions applied to the margin around the edges of the two superimposed laminate sections.

A present invention air freshener device can be produced in high volume from relatively inexpensive plastic materials. After usage, the device qualifies for disposal as a non-hazardous solid waste.

The present invention also contemplates an integrated combination of a FIG. 1 type air freshener device and a dispensing holder structure. The FIG. 1 device then functions as a replaceable refill cartridge.

### Industrial Applicability

The invention is an air freshener dispenser device useful to scent the air in a room of a dwelling, an automobile, or any other appropriate space. Stated as a method of manufacture, the invention is directly applicable to the practical manufacture of such useful devices.

## Claims

1. An air freshener dispenser device (10) which is a plastic envelope assembly comprising:
(a) an elongated thin membrane (14) which is juxtapositioned coextensively with a second elongated thin membrane (14); wherein the two membranes have dimensional correspondence, and are bonded along a peripheral margin (13) that frames a central section of opposing membrane surfaces which form a reservoir enclosure; and wherein each membrane is permeable to a volatile medium in the reservoir enclosure;
(b) a volatile air freshener medium (18) which is contained within the reservoir enclosure;
(c) a thin peelable impermeable membrane (15) which is in a laminate formation with each respective permeable membrane to prevent volatilization of the air freshener ingredient through the permeable membrane from the reservoir enclosure; and
(d) a thin semi-rigid strip (16) which is situated unattached within the reservoir enclosure, and has dimensions which closely approximate the area dimensions of the reservoir enclosure; wherein the strip functions as a stiffening means which imparts semi-rigidity to the dispenser article, and
wherein the strip structure has at least one aperture (12) which provides free access between the inner surfaces of the two permeable membranes, wherein the semi-rigid strip is a molded thermoplastic structure.

2. A dispenser device in accordance with claim 1 wherein the permeable membrane is a polyvinyl thin film.

3. A dispenser device in accordance with claim 1 wherein the impermeable membrane is an aluminum foil or nylon film.

4. A dispenser device in accordance with claim 1 wherein the volatile air freshener is a fragrance in a liquid, gel or crystalline form.

5. A dispenser device in accordance with claim 1 wherein the respective permeable membranes have transparency, and the content of a volatile air freshener medium in the reservoir enclosure is visible.

6. A dispenser device in accordance with claim 1 wherein the semi-rigid strip is a molded polyethylene or polypropylene structure.

7. A dispenser device in accordance with claim 1 wherein the respective permeable membranes have transparency, and the semi-rigid strip in the reservoir enclosure has multiple apertures which form a design pattern.

8. A dispenser device in accordance with claim 1 wherein the outer impermeable membrane is imprinted with a logo design.

9. A dispenser device in accordance with claim I which is incorporated in a dispenser housing structure, and functions as a replaceable refill cartridge.

10. A method for the manufacture of a claim 1 type of air freshener dispenser device, which comprises (1) providing two elongated thin membrane laminates of dimensional correspondence and with juxtapositional surfaces; wherein each laminate is composed of an inner vapor-permeable thin film, and a coextensive outer vapor-impermeable thin film; and wherein the two membrane laminates are bonded along a peripheral margin on three sides of the membrane laminates, thereby framing a central section of opposing membrane laminate surfaces and forming a reservoir enclosure; (2) inserting a thin semi-rigid strip through the unbonded open side of the two proximate membrane laminates, and into the reservoir enclosure; wherein the strip has dimensions which closely approximate the area dimensions of the reservoir enclosure; and wherein the strip structure has at least one aperture which provides free access between the inner surfaces of the two membrane laminates; and wherein the semi-rigid strip is a molded thermoplastic structure; (3) charging the reservoir enclosure with a volatile air freshener medium through the unbonded open side of the membrane laminates; and (4) bonding the open side of the membrane laminates to seal the volatile air freshener medium within the reservoir enclosure.

## Patentansprüche

1. Ausgabevorrichtung (10) für Luf tauffrischer, bei der es sich um eine Kunststoffumhüllung handelt mit:
(a) einer langgestreckten dünnen Membran (14), die deckungsleich mit einer zweiten langgestreckten dünnen Membran (14) und dieser gegenüber angeordnet ist, welche beiden Membranen in den Abmessungen einander entsprechen und entlang eines Umfangsrandes (13) miteinander verbunden sind, der einen mittigen Bereich einander gegenüberliegender Membranflächen einrahmt, die eine Reservoirumhüllung bilden, wobei die Membranen jeweils für ein flüchtiges Mittel in der Reservoirumhüllung durchlässig sind;
(b) einem flüchtigen Luftauffrischungsmittel (18), das in der Reservoirumhüllung enthalten ist;
(c) einer dünnen abziehbaren undurchlässigen Membran (15), die mit den durchlässigen Membranen zu einem Laminatgebilde zusammengefasst ist, um ein Verflüchtigen des Luftauffrischers aus der Reservoirumhüllung durch die durchlässige Membran hindurch zu verhindern; und
(d) einem dünnen halbstarren Streifen (16) in der Reservoirumhüllung, der nicht an dieser befestigt ist und Abmessungen aufweist, die den Flächenabmessungen der Reservoirumhüllung eng angenähert sind, wobei der Streifen als versteifende Einrichtung wirkt, die der Ausgabevorrichtung Halbstarre erteilt, der Streifenaufbau mindestens eine Öffnung (12) enthält die einen freien Zugang zu dem Raum zwischen den Innenflächen der beiden permeablen Membranen ermöglicht, und der halbstarre Streifen ein thermoplatisches Formteil ist.

2. Ausgabevorrichtung nach Anspruch 1, bei der die durchlässige Membran eine dünne Polyvinylfolie ist.

3. Ausgabevorrichtung nach Anspruch 1, bei der die undurchlässig Membran eine Aluminium- oder eine Nylonfolie ist.

4. Ausgabevorrichtung nach Anspruch 1, bei der der flüchtige Luftauffrischer ein Duftstoff in flüssiger, Gel- oder Kristallform ist.

5. Ausgabevorrichtung nach Anspruch 1, bei der die durchlässigen Membranen Transparenz aufweisen und der in der Reservoirumhüllung enthaltene flüchtige Luftauffrischer sichtbar ist.

6. Ausgabevorrichtung nach Anspruch 1, bei der der halbstarre Streifen ein Formteil aus Polyethylen oder Polypropylen ist.

7. Ausgabevorrichtung nach Anspruch 1, bei der die durchlässigen Membranen Transparenz aufweisen und der halbatarre Streifen in der Reservoirumhüllung mehrere Öffnungen enthält, die ein Design-Muster bilden.

8. Ausgabevorrichtung nach Anspruch 1, bei der die undurchlässige Membran außen mit einem Signet bzw. Logo bedruckt ist.

9. Ausgabevorrichtung nach Anspruch 1, die in ein Ausgabegehäuse eingesetzt ist und als austauschbare Nachfüllkartusche fungiert.

10. Verfahren zum Herstellen einer Ausgabevorrichtung für Luftauffrischer nach Anspruch 1, indem man (1) zwei langgestreckte dünne Membranlaminate bereit stellt, die in den Abmessungen einander entsprechen und einander gegenüber liegende Flächen aufweisen, wobei die Laminate jeweils aus einer inneren dampfdurchlässigen Folie und einer mit dieser deckungsgleichen äußeren dampfundurchlässigen Folie bestehen und die beiden Membranlaminate entlang eines Umfangsrands auf drei Seiten der Membranlaminate miteinander so verbunden werden, dass sie einen mittigen Bereich gegenüber liegender Laminatflächen einrahmen und eine Reservoirumhüllung bilden; (2) durch eine offene Seite der beiden aufeinander liegenden Membranlaminate in die Reservoirumhüllung einen dünnen halbstarren Streifen einsetzt, der sich in den Abmessungen den Flächenabmessungen der Reservoirumhüllung eng annähert, der mindestens eine Öffnung enthält, die einen freien Zugang zum Raum zwischen den Innenflächen der beiden Membranlaminate bietet, und der ein thermoplastisches Formteil ist; (3) durch die offene Seite der Membranlaminate hindurch die Reservoirumhüllung mit einem flüchtigen Luftauffrischer beschickt; und (4) die Membranlaminate entlang der offenen Seite miteinander verbindet, um den flüchtigen Luftauffrischer in die Reservoirumhüllung einzuschließen.

## Revendications

1. Dispositif distributeur de désodorisant d'ambiance (10) sous forme d'enveloppe plastique comprenant :
(a) une fine membrane allongée (14) qui est juxtaposée de manière coextensive à une seconde fine membrane allongée (14) ; dans lequel les deux membranes ont des dimensions correspondantes, et sont collées le long d'une marge périphérique (13) qui encadre une partie centrale de surfaces de membrane opposées qui forment un réservoir fermé ; et dans lequel chaque membrane est perméable à un milieu volatil dans le réservoir fermé ;
(b) un milieu de désodorisant d'ambiance volatil (18) qui est contenu à l'intérieur du réservoir fermé ;
(c) une fine membrane imperméable détachable (15) sous une forme stratifiée avec chaque membrane perméable respective pour empêcher la volatilisation de l'ingrédient de désodorisant d'ambiance présent dans le réservoir fermé à travers la membrane perméable ; et
(d) une fine bande semi-rigide (16) qui se trouve, non fixée, à l'intérieur du réservoir fermé, et dont les dimensions se rapprochent des dimensions de surface du réservoir fermé ; dans lequel la bande sert de moyen de durcissement qui confère une semi-rigidité à l'article du distributeur, et dans lequel la structure formant bande a au moins une ouverture (12) qui fournit un libre accès entre les surfaces intérieures des deux membranes perméables, dans lequel la bande semi-rigide est une structure thermoplastique moulée.

2. Dispositif distributeur selon la revendication 1, dans lequel la membrane perméable est un film fin de polyvinyle.

3. Dispositif distributeur selon la revendication 1, dans lequel la membrane imperméable est une feuille d'aluminium ou une feuille de nylon.

4. Dispositif distributeur selon la revendication 1, dans lequel le désodorisant d'ambiance volatil est un parfum dans un liquide, un gel ou sous forme cristalline.

5. Dispositif distributeur selon la revendication 1, dans lequel les membranes perméables respectives sont transparentes, et dans lequel le contenu d'un milieu de désodorisant d'ambiance volatil dans le réservoir fermé est visible.

6. Dispositif distributeur selon la revendication 1, dans lequel la bande semi-rigide est une structure moulée en polyéthylène ou en polypropylène.

7. Dispositif distributeur selon la revendication 1, dans lequel les membranes perméables respectives sont transparentes, et la bande semi-rigide dans le réservoir fermé a plusieurs ouvertures qui forment un motif.

8. Dispositif distributeur selon la revendication 1, dans lequel un logo est imprimé sur la membrane imperméable extérieure.

9. Dispositif distributeur selon la revendication 1 qui est intégré dans une structure de logement de distributeur, et sert de cartouche rechargeable remplaçable.

10. Procédé de fabrication d'un dispositif distributeur de désodorisant d'ambiance du type de la revendication 1, qui comprend les étapes consistant à (1) fournir deux stratifiés de fines membranes allongées de dimensions correspondantes et avec des surfaces juxtaposées : dans lequel chaque stratifié est constitué d'un fin film intérieur perméable à la vapeur et d'un fin film coextensif extérieur imperméable à la vapeur ; et dans lequel les deux stratifiés de membrane sont collés le long d'une marge périphérique sur les trois côtés des stratifiés de membrane, encadrant ainsi une partie centrale de surfaces de stratifiés de membrane opposées et formant un réservoir fermé ; (2) insérer une fine bande semi-rigide à travers le côté ouvert non collé des deux stratifiés de membrane rapprochés, et dans le réservoir fermé ; dans lequel la bande a des dimensions qui se rapprochent des dimensions de surface du réservoir fermé ; et dans lequel la structure de bande a au moins une ouverture qui fournit un libre accès entre les surfaces intérieures des deux stratifiés de membrane ; et dans lequel la bande semi-rigide est une structure thermoplastique moulée ; (3) remplir le réservoir fermé d'un milieu de désodorisant d'ambiance volatil par le côté ouvert non collé des stratifiés de membrane ; et (4) coller la partie ouverte des stratifiés de membrane pour enfermer le milieu de désodorisant d'ambiance volatil dans.le réservoir fermé.
